# EUROPÄISCHE PATENTSCHRIFT

(11) **EP 2 274 272 B1**
(45) Veröffentlichungstag und Bekanntmachung des Hinweises auf die Patenterteilung: **13.08.2014**
(21) Anmeldenummer: 09727925.1
(22) Anmeldetag: 18.03.2009
(51) Int. Cl.: C07C 231/02, C07C 233/18

(54) **KONTINUIERLICHES VERFAHREN ZUR HERSTELLUNG VON FETTSÄUREALKANOLAMIDEN**
CONTINUOUS METHOD FOR PRODUCING FATTY ACID ALKANOL AMIDES
PROCÉDÉ CONTINU DE PRÉPARATION D'ALCANOLAMIDES D'ACIDES GRAS

(30) Priorität: 04.04.2008 DE 102008017214
(43) Veröffentlichungstag der Anmeldung: 19.01.2011
(73) Patentinhaber: Clariant Finance (BVI) Limited, Road Town, Tortola (VG)
(72) Erfinder: KRULL, Matthias, 55296 Harxheim (DE); MORSCHHÄUSER, Roman, 55122 Mainz (DE)
(74) Vertreter: Mikulecky, Klaus
(86) Internationale Anmeldenummer: PCT/EP2009/001985
(87) Internationale Veröffentlichungsnummer: WO 2009/121485

(56) Entgegenhaltungen:
- WO-A-2008/043493
- US-A- 6 017 426
- MARTINEZ-PALOU R ET AL: "SYNTHESIS OF LONG CHAIN 2-ALKYL-1-(2-HYDROXYETHYL)-2-IMIDAZOLINES UNDER MICROWAVE IN SOLVENT-FREE CONDITIONS" SYNLETT, GEORG THIEME VERLAG, DE, Nr. 12, 1. Januar 2003 (2003-01-01), Seiten 1847-1849, XP002466232 ISSN: 0936-5214
- CABLEWSKI T ET AL: "DEVELOPMENT AND APPLICATION OF A CONTINUOUS MICROWAVE REACTOR FOR ORGANIC SYNTHESIS" JOURNAL OF ORGANIC CHEMISTRY, AMERICAN CHEMICAL SOCIETY, EASTON.; US, Bd. 59, 1. Januar 1994 (1994-01-01), Seiten 3408-3412, XP000198783 ISSN: 0022-3263
- GLASNOV ET AL: "Microwave-assisted synthesis under continuous-flow conditions" MACROMOLECULAR RAPID COMMUNICATIONS, 28(4), 395 410 CODEN: MRCOE3; ISSN: 1022-1336,, 1. Januar 2007 (2007-01-01), XP002529633

## Beschreibung

Fettsäurederivate, die funktionelle Gruppen mit hydrophilem Charakter tragen, finden weit verbreitete Verwendung als oberflächenaktive Substanzen. Eine wichtige Klasse derartiger oberflächenaktiver Substanzen sind nichtionische Amphiphile, die in großem Ausmaß beispielsweise als Emulgatoren, Korrosionsschutzmittel, Kühlschmiermittel in der Metallbearbeitung, als Lubricity-Additive in der Mineralölindustrie, als Antistatika für Polyolefine wie auch als Rohstoffe für die Herstellung von Waschmitteln, Reinigungskonzentraten, Detergentien, Kosmetika und Pharmazeutika eingesetzt werden.

Von besonderem Interesse sind dabei insbesondere Fettsäurealkanolamide, die mindestens einen über eine Amidgruppe verknüpften Alkylrest tragen, der seinerseits mit mindestens einer hydrophilen Charakter verleihenden Hydroxylgruppe substituiert ist. Diese kann vor der eigentlichen Verwendung auch weiter derivatisiert werden, beispielsweise durch Umsetzung mit Alkylenoxiden wie Ethylen-, Propylen- oder Butylenoxid bzw. durch Oxidation mit geeigneten Oxidationsmitteln. Derartige Amide weisen eine gegenüber entsprechenden Estern stark erhöhte Hydrolysestabilität auf.

Bei der technischen Herstellung von Fettsäurealkanolamiden ist man dabei bisher auf kostenintensive und/oder langwierige Herstellverfahren angewiesen, um eine kommerziell interessante Ausbeute zu erzielen. Die gängigen Herstellverfahren erfordern aktivierte Carbonsäurederivate wie beispielsweise Säureanhydride, Säurehalogenide wie Säurechloride oder Ester, die mit Hydroxylgruppen tragenden Aminen, hier im Folgenden als Alkanolamine bezeichnet, umgesetzt werden bzw. eine in-situ-Aktivierung der Reaktanden durch den Einsatz von Kupplungsreagentien wie beispielsweise N,N'-Dicyclohexylcarbodiimid. Bei diesen Herstellverfahren entstehen zum Teil große Mengen unerwünschter Nebenprodukte wie Alkohole, Säuren und Salze, die vom Produkt abgetrennt und entsorgt werden müssen. Aber auch die in den Produkten verbleibenden Reste dieser Hilfs- und Nebenprodukte können zum Teil sehr unerwünschte Effekte bewirken. So führen beispielsweise Halogenidionen wie auch Säuren zu Korrosion; Kupplungsreagentien sowie die von ihnen gebildeten Nebenprodukte sind zum Teil giftig, sensibilisierend oder auch carcinogen.

Die erstrebenswerte direkte thermische Kondensation von Carbonsäure und Alkanolamin führt zu keinen befriedigenden Resultaten, da verschiedene Nebenreaktionen die Ausbeute mindern und teilweise auch die Produkteigenschaften beeinträchtigen. Problematisch ist dabei zum einen die Bisfunktionalität der Alkanolamine, die neben der Amidbildung in erheblichem Ausmaß zu Esterbildung Anlass gibt. Da Alkanolaminester andere Eigenschaften wie beispielsweise eine deutlich geringere Hydrolysestabilität und eine geringere Löslichkeit in Wasser aufweisen, sind sie als Nebenprodukt in den meisten Anwendungen unerwünscht. Darüber hinaus führen Esteramide, bei denen sowohl Amino- wie auch Hydroxylgruppe acyliert sind, in Tensidlösungen zu unerwünschten Trübungen. Der Esteranteil kann zwar zumindest teilweise durch thermisches Behandeln in Amide umgewandelt werden, wobei jedoch auf Grund der dazu erforderlichen langen Reaktionszeiten sehr oft Farbe und Geruch der so hergestellten Alkanolamide beeinträchtigt werden. Eine Abtrennung der Esteranteile wie auch der Esteramidanteile ist aber auf Grund der meist sehr ähnlichen physikalischen Eigenschaften nur schwer oder gar nicht möglich. Als weitere unerwünschte Nebenreaktionen werden beispielsweise eine Decarboxylierung der Carbonsäure und Oxidations- wie auch Eliminierungsreaktionen der Aminogruppe während des zur Erzielung hoher Umsätze erforderlichen langen Erhitzens beobachtet. In der Regel führen diese Nebenreaktionen zum Beispiel durch Oxidation des Amins zu gefärbten Nebenprodukten und es ist nicht möglich, insbesondere für kosmetische Anwendungen gewünschte farblose Produkte mit Hazen-Farbzahlen (gemäß DIN/ISO 6271) von beispielsweise weniger als 250 herzustellen. Letzteres erfordert zusätzliche Verfahrensschritte wie beispielsweise das Bleichen, das aber seinerseits den Zusatz weiterer Hilfsstoffe erfordert und oftmals zu einer ebenso unerwünschten Beeinträchtigung des Geruchs der Amide oder zu unerwünschten Nebenprodukten wie Peroxiden und deren Abbauprodukten führt.

Ein neuerer Ansatz zur Synthese von Amiden ist die durch Mikrowellen unterstützte Umsetzung von Carbonsäuren und Aminen zu Amiden.

So offenbaren Gelens et al., Tetrahedron Letters 2005, 46(21), 3751-3754, eine Vielzahl an Amiden, die unter Zuhilfenahme von Mikrowellenstrahlung synthetisiert wurden. Darunter findet sich auch Benzoesäuremonoethanolamid, das mit einer Ausbeute von 66 % erhalten wird. Die Synthesen erfolgten in 10 ml-Gefäßen.

Massicot et al., Synthesis 2001 (16), 2441-2444 beschreiben die Synthese von Diamiden der Weinsäure im mmol-Maßstab. Bei der Amidierung mit Ethanolamin wird eine 68 %ige Ausbeute an Diamid erzielt.

R. Martinez-Palou et al., SYNLETT (2003), 1847 - 1849 lehrt ein Verfahren zur Synthese von langkettigen 2-Alkyl-1-(2-hydroxyethyl)-2-imidazol-Verbindungen und deren Amid-Vorläufermolekülen. Dabei wird Aminoethylethanolamin mit verschiedenen Fettsäuren ohne Verwendung von Lösungsmitteln unter Mikrowellenbestrahlung und Zugabe von CaO miteinander kondensiert.
EP-A-0 884 305 offenbart die Amidierung von 2-Amino-octadecandiol-1,3 mit 2-Hydroxystearinsäure unter Mikrowellenbestrahlung im mmol-Maßstab, wobei Ceramide mit einer Ausbeute von ca. 70 % entstehen.

Das Scale-Up derartiger mikrowellenunterstützter Reaktionen aus dem Labor in einen technischen Maßstab und damit die Entwicklung von Anlagen, die für eine Produktion von mehreren Tonnen wie beispielsweise mehreren zehn, mehreren hundert oder mehreren tausend Tonnen pro Jahr mit für großtechnische Anwendungen interessanten Raum-Zeit-Ausbeuten geeignet sind, konnte bisher jedoch nicht realisiert werden. Ursache dafür ist zum einen die üblicherweise auf einige Millimeter bis wenige Zentimeter begrenzte Eindringtiefe von Mikrowellen in das Reaktionsgut, was insbesondere in Batch-Verfahren durchgeführte Reaktionen auf kleine Gefäße beschränkt oder in gerührten Reaktoren zu sehr langen Reaktionszeiten führt. Einer für die Bestrahlung großer Substanzmengen mit Mikrowellen wünschenswerten Erhöhung der Feldstärke sind insbesondere in den bisher bevorzugt zum Scale-Up chemischer Reaktionen eingesetzten Multimode-Geräten durch dann auftretende Entladungsvorgänge und Plasmabildung enge Grenzen gesetzt. Weiterhin bereitet die zu lokalen Überhitzungen des Reaktionsgutes führende, durch mehr oder weniger unkontrollierte Reflektionen der in den Mikrowellenofen eingestrahlten Mikrowellen an dessen Wänden und dem Reaktionsgut verursachte Inhomogenität des Mikrowellenfeldes in den üblicherweise eingesetzten Multimode-Mikrowellengeräten Probleme bei der Maßstabsvergrößerung. Zudem bereitet der sich während der Reaktion oftmals ändernde Mikrowellen-Absorptionskoeffizient des Reaktionsgemischs Schwierigkeiten hinsichtlich einer sicheren und reproduzierbaren Reaktionsführung.

C. Chen et al., J. Chem. Soc., Chem. Commun., 1990, 807 - 809, beschreiben einen kontinuierlichen Labor-Mikrowellenreaktor, bei dem das Reaktionsgut durch eine in einem Mikrowellenofen montierte Teflon-Rohrschlange geführt wird. Ein ähnlicher kontinuierlicher Labor-Mikrowellenreaktor wird von Cablewski et al., J. Org. Chem. 1994, 59, 3408-3412 zur Durchführung verschiedenster chemischer Reaktionen beschrieben. In beiden Fällen erlaubt die im Multimode betriebene Mikrowelle jedoch kein Up-Scaling in den großtechnischen Bereich. Ihr Wirkungsgrad bezüglich der Mikrowellenabsorption des Reaktionsguts ist auf Grund der in Multimode-Mikrowellenapplikatoren auf den Applikatorraum mehr oder weniger homogen verteilten und nicht auf die Rohrschlange fokussierten Mikrowellenenergie niedrig. Eine starke Erhöhung der eingestrahlten Mikrowellenleistung führt zu unerwünschten Plasma- Entladungen. Weiterhin machen die als Hot-Spots bezeichneten, sich zeitlich verändernden räumlichen Inhomogenitäten des Mikrowellenfeldes eine sichere und reproduzierbare Reaktionsführung im großen Maßstab unmöglich.

Weiterhin sind Monomode- bzw. Singlemode-Mikrowellenapplikatoren bekannt, bei denen mit einem einzigen Wellen-Modus gearbeitet wird, der sich in nur einer Raumrichtung ausbreitet und durch exakt dimensionierte Wellenleiter auf das Reaktionsgefäß fokussiert wird. Diese Geräte erlauben zwar höhere lokale Feldstärken, sind bisher aber auf Grund der geometrischen Anforderungen (z. B. ist die Intensität des elektrischen Feldes an seinen Wellenbergen am größten und geht an den Knotenpunkten gegen Null) bisher auf kleine Reaktionsvolumina (≤ 50 ml) im Labormaßstab beschränkt.

Es wurde daher ein Verfahren zur Herstellung von Fettsäurealkanolamiden gesucht, bei dem Fettsäure und Alkanolamin auch im technischen Maßstab unter Mikrowellenbestrahlung direkt zum Alkanolamid umgesetzt werden können. Dabei sollen möglichst hohe, das heißt bis zu quantitative Umsetzungsraten erzielt werden. Insbesondere soll dabei der Anteil an Nebenprodukten wie Alkanolaminestern und Esteramiden möglichst niedrig sein. Das Verfahren soll weiterhin eine möglichst energiesparende Herstellung der Fettsäurealkanolamide ermöglichen, das heißt, die eingesetzte Mikrowellenleistung soll möglichst quantitativ vom Reaktionsgut absorbiert werden und das Verfahren somit einen hohen energetischen Wirkungsgrad bieten. Die Alkanolamide sollen ferner eine möglichst geringe Eigenfärbung aufweisen. Zudem soll das Verfahren eine sichere und reproduzierbare Reaktionsführung gewährleisten.

Überraschenderweise wurde gefunden, dass sich Fettsäurealkanolamide durch direkte Umsetzung von Fettsäuren mit Alkanolaminen in einem kontinuierlichen Verfahren durch nur kurzzeitiges Erhitzen mittels Bestrahlung mit Mikrowellen in einem Reaktionsrohr, dessen Längsachse sich in der Ausbreitungsrichtung der Mikrowellen eines Monomode-Mikrowellenapplikators befindet, in technisch relevanten Mengen herstellen lassen. Dabei wird die in den Mikrowellenapplikator eingestrahlte Mikrowellenenergie praktisch quantitativ vom Reaktionsgut absorbiert. Das erfindungsgemäße Verfahren besitzt zudem eine hohe Sicherheit bei der Durchführung und bietet eine hohe Reproduzierbarkeit der eingestellten Reaktionsbedingungen. Die nach dem erfindungsgemäßen Verfahren hergestellten Alkanolamide enthalten keine bzw. nur unwesentliche Anteile an Alkanolaminestern und Esteramiden. Sie zeigen eine im Vergleich zu nach konventionellen Herstellverfahren ohne zusätzliche Verfahrensschritte nicht zugängliche hohe Reinheit und niedrige Eigenfärbung.

Gegenstand der Erfindung ist ein kontinuierliches Verfahren zur Herstellung von Fettsäurealkanolamiden, indem mindestens eine Fettsäure der Formel I

R³-COOH (I)

worin R³ für einen gegebenenfalls substituierten aliphatischen Kohlenwasserstoffrest mit 5 bis 50 C-Atomen steht,
mit mindestens einem Alkanolamin der Formel II

HNR¹R² (II)

worin
- R¹: für einen mindestens eine Hydroxylgruppe tragenden Kohlenwasserstoffrest mit 1 bis 50 Kohlenstoffatomen und
- R²: für Wasserstoff, R¹ oder einen Kohlenwasserstoffrest mit 1 bis 50 C-Atomen stehen,
zu einem Ammoniumsalz umgesetzt wird und dieses Ammoniumsalz nachfolgend unter Mikrowellenbestrahlung in einem Reaktionsrohr, dessen Längsachse sich in der Ausbreitungsrichtung der Mikrowellen eines Monomode-Mikrowellenapplikators befindet, zum Fettsäurealkanolamid umgesetzt wird,
wobei die Bestrahlung des Salzes mit Mikrowellen in einem weitgehend mikrowellentransparenten Reaktionsrohr innerhalb eines mit einem Mikrowellengenerator über Wellenleiter verbundenen Hohlleiters erfolgt.

Fettsäurealkanolamide, mit einem Gehalt an Aminoestern und Esteramiden von weniger als 5 mol-%, sind herstellbar durch Umsetzung mindestens einer Fettsäure der Formel I

R³-COOH (I)

worin R³ für einen gegebenenfalls Substituierten aliphatischen Kohlenwasserstoffrest mit 5 bis 50 C-Atomen steht,
mit mindestens einem Alkanolamin der Formel

HNR¹R² (II)

worin
- R¹: für einen mindestens eine Hydroxylgruppe tragenden Kohlenwasserstoffrest mit 1 bis 50 Kohlenstoffatomen und
- R²: für Wasserstoff, R¹ oder einen Kohlenwasserstoffrest mit 1 bis 50 C-Atomen stehen,
zu einem Ammoniumsalz und nachfolgende Umsetzung dieses Ammoniumsalzes zum Fettsäurealkanolamid unter Mikrowellenbestrahlung in einem Reaktionsrohr, dessen Längsachse sich in der Ausbreitungsrichtung der Mikrowellen eines Monomode-Mikrowellenapplikators befindet.

Als Fettsäuren der Formel I sind allgemein Verbindungen geeignet, die mindestens eine Carboxylgruppe an einem gegebenenfalls substituierten aliphatischen Kohlenwasserstoffrest mit 5 bis 50 C-Atomen besitzen. In einer bevorzugten Ausführungsform ist der aliphatische Kohlenwasserstoffrest ein unsubstituierter Alkyl- oder Alkenylrest. In einer weiteren bevorzugten Ausführungsform ist der aliphatische Kohlenwasserstoffrest ein substituierter Alkyl- oder Alkenylrest, der einen oder mehrere wie beispielsweise zwei, drei, vier oder mehr weitere Substituenten trägt. Geeignete Substituenten sind beispielsweise Halogenatome, halogenierte Alkylreste, C₁-C₅-Alkoxy- wie beispielsweise Methoxy-, Poly(C₁-C₅-Alkoxy)-, Poly(C₁-C₅-Alkoxy)alkyl-, Carboxyl-, Ester-, Amid-, Cyano-, Nitril-, Nitro-, Sulfonsäure- und/oder Arylgruppen mit 5 bis 20 Kohlenstoffatomen wie beispielsweise Phenylgruppen mit der Maßgabe, dass diese unter den Reaktionsbedingungen stabil sind und keine Nebenreaktionen wie beispielsweise Eliminierungsreaktionen eingehen. Die C₅-C₂₀-Arylgruppen können ihrerseits wiederum Substituenten wie beispielsweise Halogenatome, halogenierte Alkylreste, C₁-C₂₀-Alkyl-, C₂-C₂₀-Alkenyl-, C₁-C₅-Alkoxy- wie beispielsweise Methoxy-, Ester-, Amid-, Cyano-, Nitril-, und/oder Nitrogruppen tragen. Der aliphatische Kohlenwasserstoffrest R³ trägt jedoch höchstens so viele Substituenten wie er Valenzen hat. In einer speziellen Ausführungsform trägt der aliphatische Kohlenwasserstoffrest R³ weitere Carboxylgruppen. So ist das erfindungsgemäße Verfahren ebenso zur Amidierung von Polycarbonsäuren mit beispielsweise zwei, drei, vier oder mehr Carboxylgruppen geeignet. Bei der Umsetzung von Polycarbonsäuren mit primären Aminen gemäß dem erfindungsgemäßen Verfahren können auch Imide entstehen.

Besonders bevorzugt sind dabei Fettsäuren (I), die einen aliphatischen Kohlenwasserstoffrest mit 6 bis 30 C-Atomen und insbesondere mit 7 bis 24 C-Atomen wie beispielsweise mit 8 bis 20 C-Atomen tragen. Sie können natürlichen oder synthetischen Ursprungs sein. Der aliphatische Kohlenwasserstoffrest kann auch Heteroatome wie beispielsweise Sauerstoff, Stickstoff, Phosphor und/oder Schwefel enthalten, bevorzugt jedoch nicht mehr als ein Heteroatom pro 3 C-Atome.

Die aliphatischen Kohlenwasserstoffreste können linear, verzweigt oder zyklisch sein. Die Carboxylgruppe kann an einem primären, sekundären oder tertiären C-Atom gebunden sein. Bevorzugt ist sie an einem primären C-Atom gebunden. Die Kohlenwasserstoffreste können gesättigt oder ungesättigt sein. Ungesättigte Kohlenwasserstoffreste enthalten eine oder mehrere C=C-Doppelbindungen und bevorzugt eine, zwei oder drei C=C-Doppelbindungen. Bevorzugt befindet sich keine Doppelbindung in α,β-Position zur Carboxylgruppe. So hat sich das erfindungsgemäße Verfahren besonders zur Herstellung von Alkanolamiden mehrfach ungesättigter Fettsäuren bewährt, da die Doppelbindungen der ungesättigten Fettsäuren unter den Reaktionsbedingungen des erfindungsgemäßen Verfahrens nicht angegriffen werden. Bevorzugte zyklische aliphatische Kohlenwasserstoffreste besitzen mindestens einen Ring mit vier, fünf, sechs, sieben, acht oder mehr Ringatomen.

Geeignete aliphatische Fettsäuren sind beispielsweise Hexan-, Cyclohexan-, Heptan-, Octan-, Decan-, Dodecan-, Tridecan-, Tetradecan-, 12-Methyltridecan-, Pentadecan-, 13-Methyltetradecan-, 12-Methyltetradecan-, Hexadecan-, 14-Methylpentadecan-, Heptadecan-, 15-Methylhexadecan-, 14-Methylhexadecan-, Octadecan-, Iso-Octadecan-, Icosan-, Docosan- und Tetracosansäure, sowie Myristolein-, Palmitolein-, Hexadecadien-, Delta-9-cis-Heptadecen-, Öl-, Petroselin-, Vaccen-, Linol-, Linolen-, Gadolein-, Gondo-, Icosadien-, Arachidon-, Cetolein-, Eruca-, Docosadien- und Tetracosensäure sowie Dodecenylbernsteinsäure, Octadecenylbernsteinsäure und deren Mischungen. Weiterhin geeignet sind aus natürlichen Fetten und Ölen wie beispielsweise Baumwollsamen-, Cocos-, Erdnuss-, Färberdistel-, Mais-, Palmkern-, Raps-, Oliven-, Senfsamen, Soja-, Sonnenblumenöl sowie Talg-, Knochen- und Fischöl gewonnene Fettsäuremischungen. Als Fettsäuren bzw. Fettsäuremischungen für das erfindungsgemäße Verfahren ebenfalls geeignet sind Tallölfettsäure sowie Harz- und Naphthensäuren.

R¹ trägt bevorzugt 2 bis 20 Kohlenstoffatome wie beispielsweise 3 bis 10 Kohlenstoffatome. Weiterhin bevorzugt steht R¹ für einen linearen oder verzweigten Alkylrest. Dieser Alkylrest kann durch Heteroatome wie Sauerstoff oder Stickstoff unterbrochen sein. R¹ trägt eine oder mehrere wie beispielsweise zwei, drei oder mehr Hydroxylgruppen. Bevorzugt befinden sich die Hydroxylgruppe bzw. Hydroxylgruppen jeweils an einem primären oder sekundären C-Atom des Kohlenwasserstoffrests. Für den Fall, dass auch R² für R¹ steht, sind Amine bevorzugt, die insgesamt höchstens 5 und insbesondere 1, 2 oder 3 Hydroxylgruppen tragen.

In einer bevorzugten Ausführungsform steht R¹ für eine Gruppe der Formel III

-(B-O)ₘ-H (III)

worin
- B: für einen Alkylenrest mit 2 bis 10 C-Atomen und
- m: für eine Zahl von 1 bis 500 steht.

Bevorzugt steht B für einen linearen oder verzweigten Alkylenrest mit 2 bis 5 C-Atomen besonders bevorzugt für einen linearen oder verzweigten Alkylenrest mit 2 oder 3 C-Atomen und insbesondere für eine Gruppe der Formel -CH₂-CH₂-, -CH₂-CH₂-CH₂- und/oder -CH(CH₃)-CH₂-.

Bevorzugt steht m für eine Zahl zwischen 2 und 300 und insbesondere für eine Zahl zwischen 3 und 100. In einer besonders bevorzugten Ausführungsform steht m für 1 oder 2. Bei Alkoxyketten mit m ≥ 3 und insbesondere mit m ≥ 5 kann es sich um eine Blockpolymerkette handeln, die alternierende Blöcke verschiedener Alkoxyeinheiten, vorzugsweise Ethoxy- und Propoxyeinheiten aufweist. Es kann sich dabei auch um eine Kette mit statistischer Abfolge der Alkoxyeinheiten oder um ein Homopolymer handeln.

In einer bevorzugten Ausführungsform steht R² für Wasserstoff, C₁-C₃₀-Alkyl, C₂-C₃₀-Alkenyl, C₅-C₁₂-Cycloalkyl, C₆-C₁₂-Aryl, C₇-C₃₀-Aralkyl oder eine heteroaromatische Gruppe mit 5 bis 12 Ringgliedern. Die Kohlenwasserstoffreste können Heteroatome wie beispielsweise Sauerstoff und/oder Stickstoff enthalten sowie gegebenenfalls Substituenten wie beispielsweise Halogenatome, halogenierte Alkylreste, Nitro-, Cyano-, Nitril-, und/oder Aminogruppen tragen. In einer weiteren bevorzugten Ausführungsform steht R² für eine Gruppe der Formel IV

-(B-O)ₘ-R⁵ (IV)

worin
- B und m: die für Formel (III) gegebenen Bedeutungen haben und
- R⁵: für einen Kohlenwasserstoffrest mit 1 bis 24 C-Atomen und insbesondere für Alkyl-, Alkenyl-, Aryl- oder Acylreste mit 1 bis 24 C-Atomen steht.

Besonders bevorzugt steht R² für Alkylreste mit 1 bis 20 C-Atomen, insbesondere mit 1 bis 8 C-Atomen und für Alkenylreste mit 2 bis 20 C-Atomen, insbesondere mit 2 bis 8 C-Atomen. Diese Alkyl- und Alkenylreste können linear, verzweigt oder zyklisch sein. Geeignete Alkyl- und Alkenylreste sind beispielsweise Methyl, Ethyl, Propyl, iso-Propyl, n-Butyl, iso-Butyl, Hexyl, Cyclohexyl, Decyl, Dodecyl, Tetradecyl, Hexadecyl, Octadecyl, iso-Stearyl und Oleyl.

In einer weiteren besonders bevorzugten Ausführungsform steht R² für einen Alkylrest mit 1 bis 4 C-Atomen wie beispielsweise Methyl oder Ethyl. In einer speziellen Ausführungsform steht R² für Wasserstoff.

Das erfindungsgemäße Verfahren eignet sich bevorzugt zur Herstellung sekundärer Fettsäurealkanolamide, das heißt zur Umsetzung von Fettsäuren mit Alkanolaminen, bei denen R¹ für einen mindestens eine Hydroxylgruppe tragenden Kohlenwasserstoffrest mit 1 bis 50 Kohlenstoffatomen und R² für Wasserstoff steht.

Das erfindungsgemäße Verfahren eignet sich besonders bevorzugt zur Herstellung tertiärer Fettsäurealkanolamide, das heißt zur Umsetzung von Fettsäuren mit Alkanolaminen, bei denen R¹ für einen mindestens eine Hydroxylgruppe tragenden Kohlenwasserstoffrest mit 1 bis 50 Kohlenstoffatomen und R² für einen Kohlenwassserstoffrest mit 1 bis 50 C-Atomen oder einen mindestens eine Hydroxylgruppe tragenden Kohlenwasserstoffrest mit 1 bis 50 Kohlenstoffatomen steht. Dabei können die Bedeutungen für R¹ und R² gleich oder verschieden sein. In einer besonders bevorzugten Ausführungsform sind die Bedeutungen für R¹ und R² dabei gleich.

Beispiele für geeignete Alkanolamine sind Aminoethanol, 3-Amino-1-propanol, Isopropanolamin, N-Methylaminoethanol, N-Ethylaminoethanol, N-Butylethanolamin, N-Methylisopropanolamin, 2-(2-Aminoethoxy)ethanol, 2-Amino-2-methyl-1-propanol, 3-Amino-2,2-dimethyl-1-propanol, 2-Amino-2-hydroxymethyl-1,3-propandiol, Diethanolamin, Dipropanolamin, Diisopropanolamin, Di(diethylenglykol)amin, N-(2-Aminoethyl)ethanolamin sowie Poly(ether)amine wie Poly(ethylenglykol)amin und Poly(propylenglykol)amin mit jeweils 4 bis 50 Alkylenoxideinheiten.

Das Verfahren ist insbesondere geeignet zur Herstellung von Octansäurediethanolamid, Laurinsäuremonoethanolamid, Laurinsäurediethanolamid, Laurinsäurediglykolamid, Cocosfettsäuremonoethanolamid, Cocosfettsäurediethanolamid, Cocosfettsäurediglykolamid, Stearinsäuremonoethanolamid, Stearinsäurediethanolamid, Stearinsäurediglykolamid, Tallölfettsäuremonoethanolamid, Tallölfettsäurediethanolamid und Tallölfettsäurediglykolamid.

Im erfindungsgemäßen Verfahren wird die Fettsäure bevorzugt mit einer mindestens equimolaren Mengen Alkanolamin und besonders bevorzugt mit einem Überschuss an Alkanolamin umgesetzt. Bevorzugt erfolgt die Umsetzung zwischen Alkanolamin und Fettsäure demzufolge mit molaren Verhältnissen von mindestens 1:1 und bevorzugt zwischen 100:1 und 1,001:1, besonders bevorzugt zwischen 10:1 und 1,01:1 wie beispielsweise zwischen 5 : 1 und 1,1:1, jeweils bezogen auf die Molequivalente an Carboxylgruppen und Aminogruppen im Reaktionsgemisch. Dabei werden die Carboxylgruppen praktisch quantitativ zum Amid umgesetzt. In einer speziellen Ausführungsform werden Fettsäure und Amin equimolar eingesetzt.

Die erfindungsgemäße Herstellung der Amide erfolgt durch Umsetzung von Fettsäure und Alkanolamin zum Ammoniumsalz und nachfolgender Bestrahlung des Salzes mit Mikrowellen in einem Reaktionsrohr, dessen Längsachse sich in der Ausbreitungsrichtung der Mikrowellen in einem Monomode-Mikrowellenapplikators befindet.

Die Bestrahlung des Satzes mit Mikrowellen erfolgt in einem weitgehend mikrowellentransparenten Reaktionsrohr, das sich innerhalb eines mit einem Mikrowellengenerator verbundenen Hohlleiters befindet. Bevorzugt fluchtet das Reaktionsrohr axial mit der zentralen Symmetrieachse des Hohlleiters.

Der als Mikrowellenapplikator fungierende Hohlleiter ist bevorzugt als Hohlraumresonator ausgeformt. Weiterhin bevorzugt werden die im Hohlleiter nicht absorbierten Mikrowellen an seinem Ende reflektiert. Durch Ausformung des Mikrowellenapplikators als Resonator vom Reflexionstyp werden eine lokale Erhöhung der elektrischen Feldstärke bei gleicher vom Generator zugeführter Leistung und eine erhöhte Energieausnutzung erzielt.

Der Hohlraumresonator wird bevorzugt im E₀₁ₙ-Mode betrieben, wobei n für eine ganze Zahl steht und die Anzahl der Feldmaxima der Mikrowelle entlang der zentralen Symmetrieachse des Resonators angibt. Bei diesem Betrieb ist das elektrische Feld in Richtung der zentralen Symmetrieachse des Hohlraumresonators gerichtet. Es hat im Bereich der zentralen Symmetrieachse ein Maximum und nimmt zur Mantelfläche hin auf den Wert null ab. Diese Feldkonfiguration liegt rotationssymmetrisch um die zentrale Symmetrieachse vor. Je nach der gewünschten Strömungsgeschwindigkeit des Reaktionsguts durch das Reaktionsrohr, der benötigten Temperatur und der benötigten Verweilzeit im Resonator wird die Länge des Resonators relativ zu der Wellenlänge der eingesetzten Mikrowellenstrahlung ausgewählt. Bevorzugt ist n eine ganze Zahl von 1 bis 200, besonders bevorzugt von 2 bis 100, insbesondere von 4 bis 50 und speziell von 3 bis 20 wie beispielsweise 3, 4, 5, 6, 7 oder 8.

Die Einstrahlung der Mikrowellenenergie in den als Mikrowellenapplikator fungierenden Hohlleiter kann über geeignet dimensionierte Löcher oder Schlitze erfolgen. In einer erfindungsgemäß besonders bevorzugten Ausführungsform erfolgt die Bestrahlung des Ammoniumsalzes mit Mikrowellen in einem Reaktionsrohr, das sich in einem Hohlleiter mit koaxialem Übergang der Mikrowellen befindet. Für dieses Verfahren besonders bevorzugte Mikrowelleneinrichtungen sind aus einem Hohlraumresonator, einer Koppeleinrichtung zum Einkoppeln eines Mikrowellenfeldes in den Hohlraumresonator und mit je einer Öffnung an zwei gegenüber liegenden Stirnwänden zum Hindurchführen des Reaktionsrohres durch den Resonator aufgebaut. Die Einkopplung der Mikrowellen in den Hohlraumresonator erfolgt bevorzugt über einen Koppelstift, der in den Hohlraumresonator hineinragt. Bevorzugt ist der Koppelstift als ein als Kopplungsantenne fungierendes, bevorzugt metallisches Innenleiterrohr ausgeformt. In einer besonders bevorzugten Ausführungsform ragt dieser Koppelstift durch eine der stirnseitigen Öffnungen in den Hohlraumresonator hinein. Besonders bevorzugt schließt sich das Reaktionsrohr an das Innenleiterrohr des koaxialen Übergangs an und speziell wird es durch dessen Hohlraum hindurch in den Hohlraumresonator geführt. Bevorzugt fluchtet das Reaktionsrohr axial mit einer zentralen Symmetrieachse des Hohlraumresonators, wozu der Hohlraumresonator bevorzugt je eine zentrische Öffnung an zwei gegenüber liegenden Stirnwänden zum Hindurchführen des Reaktionsrohres aufweist.

Die Einspeisung der Mikrowellen in den Koppelstift bzw. in das als Kopplungsantenne fungierende Innenleiterrohr kann beispielsweise mittels einer koaxialen Anschlussleitung erfolgen. In einer bevorzugten Ausführungsform wird das Mikrowellenfeld über einen Hohlleiter dem Resonator zugeführt, wobei das aus dem Hohlraumresonator herausragende Ende des Koppelstifts in eine Öffnung, die sich in der Wand des Hohlleiters befindet, in den Hohlleiter hineingeführt ist und von dem Hohlleiter Mikrowellenenergie entnimmt und in den Resonator koppelt.

In einer speziellen Ausführungsform erfolgt die Bestrahlung des Salzes mit Mikrowellen in einem mikrowellentransparenten Reaktionsrohr, das sich axialsymmetrisch in einem E₀₁ₙ-Rundhohlleiter mit koaxialem Übergang der Mikrowellen befindet. Dabei wird das Reaktionsrohr durch den Hohlraum eines als Kopplungsantenne fungierenden Innenleiterrohres in den Hohlraumresonator geführt. In einer weiteren bevorzugten Ausführungsform erfolgt die Bestrahlung des Salzes mit Mikrowellen in einem mikrowellentransparenten Reaktionsrohr, das durch einen E₀₁ₙ-Hohlraumresonator mit axialer Einspeisung der Mikrowellen geführt wird, wobei die Länge des Hohlraumresonators so bemessen ist, dass sich n = 2 oder mehr Feldmaxima der Mikrowelle ausbilden. In einer weiteren bevorzugten Ausführungsform erfolgt die Bestrahlung des Salzes mit Mikrowellen in einem mikrowellentransparenten Reaktionsrohr, das sich axialsymmetrisch in einem kreiszylindrischen E₀₁ₙ-Hohlraumresonator mit koaxialem Übergang der Mikrowellen befindet, wobei die Länge des Hohlraumresonators so bemessen ist, dass sich n = 2 oder mehr Feldmaxima der Mikrowelle ausbilden.

Mikrowellengeneratoren, wie beispielsweise das Magnetron, das Klystron und das Gyrotron sind dem Fachmann bekannt.

Die zur Durchführung des erfindungsgemäßen Verfahrens eingesetzten Reaktionsrohre sind bevorzugt aus weitgehend mikrowellentransparentem, hoch schmelzendem Material gefertigt. Besonders bevorzugt werden nichtmetallische Reaktionsrohre eingesetzt. Unter weitgehend mikrowellentransparent werden hier Werkstoffe verstanden, die möglichst wenig Mikrowellenenergie absorbieren und in Wärme umwandeln. Als Maß für die Fähigkeit eines Stoffes, Mikrowellenenergie zu absorbieren und in Wärme zu überführen wird oftmals der dielektrische Verlustfaktor tan δ = ε"/ε' herangezogen. Der dielektrische Verlustfaktor tan δ ist definiert als das Verhältnis aus dielektrischem Verlust ε" und Dielektrizitätskonstante ε'. Beispiele für tan δ-Werte verschiedener Materialien sind beispielsweise in D. Bogdal, Microwave-assisted Organic Synthesis, Elsevier 2005 wiedergegeben. Für erfindungsgemäß geeignete Reaktionsrohre werden Materialen mit bei 2,45 GHz und 25 °C gemessenen tan δ-Werten von unter 0,01, insbesondere unter 0,005 und speziell unter 0,001 bevorzugt. Als bevorzugte mikrowellentransparente und temperaturstabile Materialien kommen in erster Linie Werkstoffe auf mineralischer Basis wie beispielsweise Quarz, Aluminiumoxid, Zirkonoxid und ähnliches in Betracht. Auch temperaturstabile Kunststoffe wie insbesondere Fluorpolymere wie beispielsweise Teflon, und technische Kunststoffe wie Polypropylen, oder Polyaryletherketone wie beispielsweise glasfaserverstärktes Polyetheretherketon (PEEK) sind als Rohrmaterialien geeignet. Um den Temperaturbedingungen während der Reaktion zu widerstehen haben sich insbesondere mit diesen Kunststoffen beschichtete Mineralien wie Quarz oder Aluminiumoxid als Reaktormaterialien bewährt.

Für das erfindungsgemäße Verfahren besonders geeignete Reaktionsrohre haben einen Innendurchmesser von einem Millimeter bis ca. 50 cm, speziell zwischen 2 mm und 35 cm wie beispielsweise zwischen 5 mm und 15 cm. Unter Reaktionsrohren werden hier Gefäße verstanden, deren Verhältnis von Länge zu Durchmesser größer als 5, bevorzugt zwischen 10 und 100.000, besonders bevorzugt zwischen 20 und 10.000 wie beispielsweise zwischen 30 und 1.000 ist. Unter der Länge des Reaktionsrohres wird hier die Strecke des Reaktionsrohres verstanden, auf der die Mikrowellenbestrahlung erfolgt. In das Reaktionsrohr können Stromstörer und/oder andere Mischelemente eingebaut sein.

Für das erfindungsgemäße Verfahren besonders geeignete E₀₁-Hohlraumresonatoren haben bevorzugt einen Durchmesser, der mindestens der halben Wellenlänge der verwendeten Mikrowellenstrahlung entspricht. Bevorzugt beträgt der Durchmesser des Hohlraumresonators das 1,0- bis 10-fache, besonders bevorzugt das 1,1- bis 5-fache und insbesondere das 2,1- bis 2,6-fache der halben Wellenlänge der verwendeten Mikrowellenstrahlung. Bevorzugt hat der E₀₁-Hohlraumresonator einen runden Querschnitt, was auch als E₀₁-Rundhohlleiter bezeichnet wird. Besonders bevorzugt hat er eine zylindrische Form und speziell eine kreiszylindrische Form.

Das Reaktionsrohr ist üblicherweise am Einlass mit einer Dosierpumpe sowie einem Manometer und am Auslass mit einer Druckhaltevorrichtung und einem Wärmetauscher versehen. Damit sind Reaktionen in einem sehr weiten Druck- und Temperaturbereich möglich.

Die Umsetzung von Amin und Fettsäure zum Ammoniumsalz kann kontinuierlich, diskontinuierlich oder auch in semi-Batch-Prozessen durchgeführt werden. So kann die Herstellung des Ammoniumsalzes in einem vorgelagerten (semi)-Batch Prozess durchgeführt werden wie beispielsweise in einem Rührbehälter. Das Ammoniumsalz wird bevorzugt in-situ erzeugt und nicht isoliert. In einer bevorzugten Ausführungsform werden die Edukte Amin und Fettsäure, beide unabhängig voneinander gegebenenfalls mit Lösemittel verdünnt, erst kurz vor dem Eintritt in das Reaktionsrohr vermischt. So hat es sich besonders bewährt, die Umsetzung von Amin und Fettsäure zum Ammoniumsalz in einer Mischstrecke vorzunehmen, aus der das Ammoniumsalz, gegebenenfalls nach Zwischenkühlung, in das Reaktionsrohr gefördert wird. Weiterhin bevorzugt werden die Edukte dem erfindungsgemäßen Verfahren in flüssiger Form zugeführt. Dazu können höher schmelzende und/oder höher viskose Edukte beispielsweise in geschmolzenem Zustand und/oder mit Lösemittel versetzt beispielsweise als Lösung, Dispersion oder Emulsion eingesetzt werden. Ein Katalysator kann, sofern eingesetzt, einem der Edukte oder auch der Eduktmischung vor dem Eintritt in das Reaktionsrohr zugesetzt werden. Auch feste, pulverförmige und heterogene Systeme können nach dem erfindungsgemäßen Verfahren umgesetzt werden, wobei lediglich entsprechende technische Vorrichtungen zum Fördern des Reaktionsgutes erforderlich sind.

Das Ammoniumsalz kann in das Reaktionsrohr entweder an dem durch das Innenleiterrohr geführte Ende eingespeist werden als auch an dem entgegen gesetzten Ende.

Durch Variation von Rohrquerschnitt, Länge der Bestrahlungszone (hierunter wird die Strecke des Reaktionsrohres verstanden, in dem das Reaktionsgut Mikrowellenstrahlung ausgesetzt ist), Fließgeschwindigkeit, Geometrie des Hohlraumresonators, der eingestrahlten Mikrowellenleistung sowie der dabei erreichten Temperatur werden die Reaktionsbedingungen so eingestellt, dass die maximale Reaktionstemperatur schnellstmöglich erreicht wird und die Verweilzeit bei Maximaltemperatur so kurz bleibt, dass so wenig Neben- oder Folgereaktionen wie möglich auftreten. Das Reaktionsgut kann zur Vervollständigung der Reaktion, gegebenenfalls nach Zwischenkühlung, mehrfach das Reaktionsrohr durchlaufen. Vielfach hat sich bewährt, wenn das Reaktionsprodukt unmittelbar nach Verlassen des Reaktionsrohres z. B. durch Mantelkühlung oder Entspannung abgekühlt wird. Bei langsamer verlaufenden Reaktionen hat es sich oftmals bewährt, das Reaktionsprodukt nach Verlassen des Reaktionsrohres noch eine gewisse Zeit bei Reaktionstemperatur zu halten.

Die Vorteile des erfindungsgemäßen Verfahrens liegen in einer sehr gleichmäßigen Bestrahlung des Reaktionsgutes im Zentrum eines symmetrischen Mikrowellenfeldes innerhalb eines Reaktionsrohres, dessen Längsachse sich in der Ausbreitungsrichtung der Mikrowellen eines Monomode-Mikrowellenapplikators und insbesondere innerhalb eines E₀₁-Hohlraumresonators beispielsweise mit koaxialem Übergang befindet. Dabei erlaubt das erfindungsgemäße Reaktordesign die Durchführung von Reaktionen auch bei sehr hohen Drücken und/oder Temperaturen. Durch Temperatur- und/oder Druckerhöhung wird eine deutliche Steigerung von Umsetzungsgrad und Ausbeute auch gegenüber bekannten Mikrowellenreaktoren beobachtet, ohne dass es dabei zu unerwünschten Nebenreaktionen und/oder Verfärbungen kommt. Überraschenderweise wird dabei wird ein sehr hoher Wirkungsgrad bei der Ausnutzung der in den Hohlraumresonator eingestrahlten Mikrowellenenergie erzielt, der üblicherweise über 50 %, oftmals über 80 %, teilweise über 90 % und in speziellen Fällen über 95 % wie beispielsweise über 98 % der eingestrahlten Mikrowellenleistung liegt und somit ökonomische wie auch ökologische Vorteile gegenüber konventionellen Herstellverfahren wie auch gegenüber Mikrowellenverfahren des Standes der Technik bietet.

Das erfindungsgemäße Verfahren erlaubt zudem eine kontrollierte, sichere und reproduzierbare Reaktionsführung. Da das Reaktionsgut im Reaktionsrohr parallel zur Ausbreitungsrichtung der Mikrowellen bewegt wird, werden bekannte Überhitzungsphänomene durch unkontrollierbare Feldverteilungen, die zu lokalen Überhitzungen durch wechselnde Intensitäten des Feldes beispielsweise in Wellenbergen und Knotenpunkten führen, durch die Fließbewegung des Reaktionsgutes ausgeglichen. Die genannten Vorteile erlauben es auch, mit hohen Mikrowellenleistungen von beispielsweise mehr als 10 kW oder mehr als 100 kW zu arbeiten und somit in Kombination mit einer nur kurzen Verweilzeit im Hohlraumresonator große Produktionsmengen von 100 und mehr Tonnen pro Jahr in einer Anlage zu bewerkstelligen.

Dabei war es besonders überraschend, dass trotz der im kontinuierlich durchflossenen Strömungsrohr nur sehr kurzen Verweildauer des Ammoniumsalzes im Mikrowellenfeld eine sehr weitgehende Amidierung mit Umsätzen im allgemeinen von über 80 %, oftmals auch über 90 % wie beispielsweise über 95 % bezogen auf die im Unterschuss eingesetzte Komponente stattfindet, ohne Bildung nennenswerter Mengen an Nebenprodukten. Bei einer entsprechenden Umsetzung dieser Ammoniumsalze in einem Strömungsrohr gleicher Dimensionierung unter thermischer Mantelheizung werden zur Erzielung geeigneter Reaktionstemperaturen extrem hohe Wandtemperaturen benötigt, die zur Bildung gefärbter Spezies führten, aber bei gleichem Zeitintervall nur geringfügige Amidbildung bewirken.

Bevorzugt wird der durch die Mikrowellenbestrahlung bedingte Temperaturanstieg beispielsweise durch Regelung der Mikrowellenintensität, der Durchflussgeschwindigkeit und/oder durch Kühlung des Reaktionsrohres, beispielsweise durch einen Stickstoffstrom, auf maximal 500 °C begrenzt. Besonders bewährt hat sich die Durchführung der Umsetzung bei Temperaturen zwischen 150 und maximal 400 °C und speziell zwischen 180 und maximal 300 °C wie beispielsweise bei Temperaturen zwischen 200 und 270 °C.

Die Dauer der Mikrowellenbestrahlung hängt von verschiedenen Faktoren wie beispielsweise der Geometrie des Reaktionsrohres, der eingestrahlten Mikrowellenenergie, der speziellen Reaktion und dem gewünschten Umsetzungsgrad ab. Üblicherweise wird die Mikrowellenbestrahlung über einen Zeitraum von weniger als 30 Minuten, bevorzugt zwischen 0,01 Sekunden und 15 Minuten, besonders bevorzugt zwischen 0,1 Sekunden und 10 Minuten und insbesondere zwischen einer Sekunde und 5 Minuten wie beispielsweise zwischen 5 Sekunden und 2 Minuten vorgenommen. Die Intensität (Leistung) der Mikrowellenstrahlung wird dabei so eingestellt, dass das Reaktionsgut beim Verlassen des Hohlraumresonators die gewünschte Maximaltemperatur hat. In einer bevorzugten Ausführungsform wird das Umsetzungsprodukt direkt nach Beendigung der Mikrowellenbestrahlung möglichst schnell auf Temperaturen unterhalb 120 °C, bevorzugt unterhalb 100 °C und speziell unterhalb 60 °C abgekühlt.

Bevorzugt wird die Umsetzung bei Drücken zwischen 0,01 und 500 bar und besonders bevorzugt zwischen 1 bar (Atmosphärendruck) und 150 bar und speziell zwischen 1,5 bar und 100 bar wie beispielsweise zwischen 3 bar und 50 bar durchgeführt. Besonders bewährt hat sich das Arbeiten unter erhöhtem Druck, wobei oberhalb des Siedepunkts (bei Normaldruck) der Edukte bzw. Produkte, des gegebenenfalls anwesenden Lösemittels und/oder oberhalb des während der Reaktion gebildeten Reaktionswassers gearbeitet wird. Besonders bevorzugt wird der Druck so hoch eingestellt, dass das Reaktionsgemisch während der Mikrowellenbestrahlung im flüssigen Zustand verbleibt und nicht siedet.

Zur Vermeidung von Nebenreaktionen und zur Herstellung von möglichst reinen Produkten hat es sich bewährt, Edukte und Produkte in Gegenwart eines inerten Schutzgases wie beispielsweise Stickstoff, Argon oder Helium zu handhaben.

In einer bevorzugten Ausführungsform wird zur Beschleunigung bzw. zur Vervollständigung der Reaktion in Gegenwart von dehydratisierenden Katalysatoren gearbeitet. Vorzugsweise arbeitet man dabei in Gegenwart eines sauren anorganischen, metallorganischen oder organischen Katalysators oder Gemischen aus mehreren dieser Katalysatoren.

Als saure anorganische Katalysatoren im Sinne der vorliegenden Erfindung sind beispielsweise Schwefelsäure, Phosphorsäure, Phosphonsäure, hypophosphorige Säure, Aluminiumsulfathydrat, Alaun, saures Kieselgel und saures Aluminiumhydroxid zu nennen. Weiterhin sind beispielsweise Aluminiumverbindungen der allgemeinen Formel Al(OR¹⁵)₃ und Titanate der allgemeinen Formel Ti(OR¹⁵)₄ als saure anorganische Katalysatoren einsetzbar, wobei die Reste R¹⁵ jeweils gleich oder verschieden sein können und unabhängig voneinander gewählt sind aus C₁-C₁₀-Alkylresten, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, sec.-Hexyl, n-Heptyl, n-Octyl, 2-Ethylhexyl, n-Nonyl oder n-Decyl, C₃-C₁₂-Cycloalkylresten, beispielsweise Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl. Bevorzugt sind die Reste R¹⁵ in Al(OR¹⁵)₃ bzw. Ti(OR¹⁵)₄ jeweils gleich und gewählt aus Isopropyl, Butyl und 2-Ethylhexyl.

Bevorzugte saure metallorganische Katalysatoren sind beispielsweise gewählt aus Dialkylzinnoxiden (R¹⁵)₂SnO, wobei R¹⁵ wie oben stehend definiert ist. Ein besonders bevorzugter Vertreter für saure metallorganische Katalysatoren ist Di-n-butylzinnoxid, das als sogenanntes Oxo-Zinn oder als Fascat^{®}-Marken kommerziell erhältlich ist.

Bevorzugte saure organische Katalysatoren sind saure organische Verbindungen mit beispielsweise Phosphatgruppen, Sulfonsäuregruppen, Sulfatgruppen oder Phosphonsäuregruppen. Besonders bevorzugte Sulfonsäuren enthalten mindestens eine Sulfonsäuregruppe und mindestens einen gesättigten oder ungesättigten, linearen, verzweigten und/oder zyklischen Kohlenwasserstoffrest mit 1 bis 40 C-Atomen und bevorzugt mit 3 bis 24 C-Atomen. Insbesondere bevorzugt sind aromatische Sulfonsäuren, speziell alkylaromatische Mono-Sulfonsäuren mit einem oder mehreren C₁-C₂₈-Alkylresten und insbesondere solche mit C₃-C₂₂-Alkylresten. Geeignete Beispiele sind Methansulfonsäure, Butansulfonsäure, Benzolsulfonsäure, p-Toluolsulfonsäure, Xylolsulfonsäure, 2-Mesitylensulfonsäure, 4-Ethylbenzolsulfonsäure, Isopropylbenzolsulfonsäure, 4-Butylbenzolsulfonsäure, 4-Octylbenzolsulfonsäure; Dodecylbenzolsulfonsäure, Didodecylbenzolsulfonsäure, Naphthalinsulfonsäure. Auch saure Ionenaustauscher können als saure organische Katalysatoren eingesetzt werden, beispielsweise Sulfonsäuregruppenhaltige Poly(styrol)-Harze, die mit etwa 2 mol-% Divinylbenzol vernetzt sind.

Besonders bevorzugt für die Durchführung des erfindungsgemäßen Verfahrens sind Borsäure, Phosphorsäure, Polyphosphorsäure und Polystyrolsulfonsäuren. Insbesondere bevorzugt sind Titanate der allgemeinen Formel Ti(OR¹⁵)₄ und speziell Titantetrabutylat und Titantetraisopropylat.

Wünscht man saure anorganische, metallorganische oder organische Katalysatoren einzusetzen, so setzt man erfindungsgemäß 0,01 bis 10 Gew.-%, bevorzugt 0,02 bis 2 Gew.-% Katalysator ein. In einer besonders bevorzugten Ausführungsform wird ohne Katalysator gearbeitet.

In einer weiteren bevorzugten Ausführungsform wird die Mikrowellenbestrahlung in Gegenwart von sauren, festen Katalysatoren durchgeführt. Dabei wird der feste Katalysator in dem gegebenenfalls mit Lösemittel versetzten Ammoniumsalz suspendiert oder vorteilhafterweise das gegebenenfalls mit Lösemittel versetzte Ammoniumsalz über einen Festbettkatalysator geleitet und Mikrowellenstrahlung ausgesetzt. Geeignete feste Katalysatoren sind beispielsweise Zeolithe, Kieselgel, Montmorillonit und (teil)vernetzte Polystyrolsulfonsäure, die gegebenenfalls mit katalytisch aktiven Metallsalzen imprägniert sein können. Geeignete saure Ionentauscher auf Basis von Polystyrolsulfonsäuren, die als Festphasenkatalysatoren eingesetzt werden können, sind beispielsweise von der Firma Rohm & Haas unter der Markenbezeichnung Amberlyst^{®} erhältlich.

Es hat sich bewährt, in Gegenwart von Lösemitteln zu arbeiten um beispielsweise die Viskosität des Reaktionsmediums abzusenken und/oder das Reaktionsgemisch, sofern es heterogen ist, zu fluidisieren. Dafür können prinzipiell alle Lösemittel eingesetzt werden, die unter den angewendeten Reaktionsbedingungen inert sind und nicht mit den Edukten bzw. den gebildeten Produkten reagieren. Ein wichtiger Faktor bei der Auswahl geeigneter Lösemittel ist deren Polarität, die einerseits die Löseeigenschaften und andererseits das Ausmaß der Wechselwirkung mit Mikrowellenstrahlung bestimmt. Ein besonders wichtiger Faktor bei der Auswahl geeigneter Lösemittel ist deren dielektrischer Verlust ε". Der dielektrische Verlust ε" beschreibt den Anteil an Mikrowellenstrahlung, der bei der Wechselwirkung einer Substanz mit Mikrowellenstrahlung in Wärme überführt wird. Letzt genannter Wert hat sich als besonders wichtiges Kriterium für die Eignung eines Lösemittels für die Durchführung des erfindungsgemäßen Verfahrens erwiesen. Besonders bewährt hat sich das Arbeiten in Lösemitteln, die eine möglichst geringe Mikrowellenabsorption zeigen und somit nur einen kleinen Beitrag zur Erwärmung des Reaktionssystems liefern. Für das erfindungsgemäße Verfahren bevorzugte Lösemittel besitzen einen bei Raumtemperatur und 2450 MHz gemessenen dielektrischen Verlust ε" von weniger als 10 und vorzugsweise weniger als 1 wie beispielsweise weniger als 0,5. Eine Übersicht über den dielektrischen Verlust verschiedener Lösemittel findet sich zum Beispiel in "Microwave Synthesis" von B. L. Hayes, CEM Publishing 2002. Für das erfindungsgemäße Verfahren geeignet sind insbesondere Lösemittel mit ε"-Werten unterhalb 10 wie N-Methylpyrrolidon, N,N-Dimethylformamid oder Aceton, und insbesondere Lösemittel mit ε"-Werten unterhalb 1. Beispiele für besonders bevorzugte Lösemittel mit ε"-Werten unterhalb 1 sind aromatische und/oder aliphatische Kohlenwasserstoffe wie beispielsweise Toluol, Xylol, Ethylbenzol, Tetralin, Hexan, Cyclohexan, Decan, Pentadecan, Dekalin sowie kommerzielle Kohlenwasserstoffgemische wie Benzinfraktionen, Kerosin, Solvent Naphtha, Shellsol^{®} AB, Solvesso^{®} 150, Solvesso^{®} 200, Exxsol^{®}, Isopar^{®} und Shellsol^{®}-Typen. Lösemittelgemische, die ε"-Werte bevorzugt unterhalb 10 und speziell unterhalb 1 aufweisen, sind für die Durchführung des erfindungsgemäßen Verfahrens gleichermaßen bevorzugt.

Prinzipiell ist das erfindungsgemäße Verfahren auch in Lösemitteln mit höheren ε"-Werten von beispielsweise 5 und höher wie insbesondere mit ε"-Werten von 10 und höher durchführbar. Die dabei beobachtete beschleunigte Aufheizung des Reaktionsgemischs erfordert jedoch besondere Maßnahmen zur Einhaltung der Maximaltemperatur.

Sofern in Gegenwart von Lösemitteln gearbeitet wird, liegt deren Anteil an der Reaktionsmischung bevorzugt zwischen 2 und 95 Gew.-%, speziell zwischen 5 und 90 Gew.-% und insbesondere zwischen 10 und 75 Gew.-% wie beispielsweise zwischen 30 und 60 Gew.-%. Besonders bevorzugt wird die Reaktion lösemittelfrei durchgeführt.

Als Mikrowellen werden elektromagnetische Strahlen mit einer Wellenlänge zwischen etwa 1 cm und 1 m und Frequenzen zwischen etwa 300 MHz und 30 GHz bezeichnet. Dieser Frequenzbereich ist prinzipiell für das erfindungsgemäße Verfahren geeignet. Bevorzugt wird für das erfindungsgemäße Verfahren Mikrowellenstrahlung mit den für industrielle, wissenschaftliche und medizinische Anwendungen freigegebenen Frequenzen verwendet wie beispielsweise mit Frequenzen von 915 MHz, 2,45 GHz, 5,8 GHz oder 27,12 GHz.

Die für die Durchführung des erfindungsgemäßen Verfahrens in den Hohlraumresonator einzustrahlende Mikrowellenleistung ist insbesondere abhängig von der Geometrie des Reaktionsrohrs und damit des Reaktionsvolumens sowie der Dauer der erforderlichen Bestrahlung. Sie liegt üblicherweise zwischen 200 W und mehreren 100 kW und insbesondere zwischen 500 W und 100 kW wie beispielsweise zwischen 1 kW und 70 kW. Sie kann über einen oder mehrere Mikrowellengeneratoren erzeugt werden.

In einer bevorzugten Ausführungsform wird die Reaktion in einem druckfesten inert-Rohr durchgeführt, wobei das sich bildende Reaktionswasser sowie gegebenenfalls Edukte und, sofern anwesend, Lösemittel zu einem Druckaufbau führen. Nach Beendigung der Reaktion kann der Überdruck durch Entspannung zur Verflüchtigung und Abtrennung von Reaktionswasser, überschüssigen Edukten sowie gegebenenfalls Lösemittel und/oder zur Abkühlung des Reaktionsprodukts verwendet werden. In einer weiteren Ausführungsform wird das gebildete Reaktionswasser nach dem Abkühlen und/oder Entspannen durch übliche Verfahren wie beispielsweise Phasentrennung, Destillation Strippen, Flashen und/oder Absorption abgetrennt.

Zur Vervollständigung der Umsetzung hat es sich in vielen Fällen bewährt, das erhaltene Rohprodukt nach Entfernen von Reaktionswasser sowie gegebenenfalls Austragen von Produkt und/oder Nebenprodukt erneut der Mikrowellenbestrahlung auszusetzen, wobei gegebenenfalls das Verhältnis der eingesetzten Reaktanden um verbrauchte oder unterschüssige Edukte zu ergänzen ist.

Üblicherweise fallen über den erfindungsgemäßen Weg hergestellte Alkanolamide in einer für die weitere Verwendung ausreichenden Reinheit an. Für spezielle Anforderungen können sie jedoch nach üblichen Reinigungsverfahren wie beispielsweise Destillation, Umkristallisation, Filtration bzw. chromatographische Verfahren weiter aufgereinigt werden.

Das erfindungsgemäße Verfahren erlaubt eine sehr schnelle, energiesparende und kostengünstige Herstellung von Fettsäurealkanolamiden in hohen Ausbeuten und mit hoher Reinheit in großtechnischen Mengen. Durch die sehr gleichmäßige Bestrahlung des Ammoniumsalzes im Zentrum des rotationssymmetrischen Mikrowellenfeldes erlaubt es eine sichere, kontrollierbare und reproduzierbare Reaktionsführung. Dabei wird durch einen sehr hohen Wirkungsgrad bei der Ausnutzung der eingestrahlten Mikrowellenenergie eine den bekannten Herstellverfahren deutlich überlegene Wirtschaftlichkeit erzielt. Bei diesem Verfahren fallen keine wesentlichen Mengen an Nebenprodukten an.

Insbesondere weisen die nach dem erfindungsgemäßen Verfahren hergestellten Alkanolamide nur einen geringen Gehalt an Alkanolaminestern wie auch an Esteramiden auf. Ihre wässrigen Lösungen sind daher klar und weisen im Gegensatz zu entsprechenden, durch thermische Kondensation hergestellte Fettsäurealkanolamide keine durch Esteramide verursachten Trübungen auf. Die Eigenfärbung der erfindungsgemäß hergestellten Amide entspricht Hazen-Farbzahlen (nach DIN/ISO 6271) von unter 200 und teilweise unter 150 wie beispielsweise unterhalb 100, wogegen nach klassischen Methoden Hazen-Farbzahlen unterhalb 250 ohne zusätzliche Verfahrensschritte nicht zugänglich sind. Da die nach dem erfindungsgemäßen Verfahren hergestellten Alkanolamide zudem verfahrensbedingt keine Reste von Kupplungsreagentien bzw. deren Folgeprodukten enthalten, können sie problemlos auch in toxikologisch sensiblen Bereichen wie beispielsweise kosmetischen und pharmazeutischen Zubereitungen eingesetzt werden.

Die erfindungsgemäß hergestellten Alkanolamide enthalten bezogen auf die Gesamtheit der vorhandenen Fettsäuren und Fettsäurederivate bevorzugt weniger als 5 mol-%, speziell weniger als 2 mol-% und insbesondere praktisch keine aus der Acylierung der Hydroxylgruppe des Alkanolamins resultierenden Ester bzw. Esteramide. Unter praktisch keine Ester und Alkanolaminester enthaltend werden Alkanolamide verstanden, deren Gesamtgehalt an Estern und Esteramiden unter 1 mol-% liegt und mit üblichen Analysenverfahren wie beispielsweise der ¹H-NMR-Spektroskopie nicht nachgewiesen werden kann.

Derartig schnelle und selektive Umsetzungen sind nach klassischen Methoden nicht zu erzielen und waren alleine durch Heizen auf hohe Temperaturen nicht zu erwarten. Die nach dem erfindungsgemäßen Verfahren hergestellten Produkte sind oftmals so rein, dass keine weiteren Auf- oder Nacharbeitungsschritte erforderlich sind.

### Beispiele

Die Umsetzungen der Ammoniumsalze unter Mikrowellenbestrahlung erfolgten in einem Keramikrohr (60 x 1 cm), das sich axialsymmetrisch in einem zylindrischen Hohlraumresonator (60 x 10 cm) befand. An einer der Stirnseiten des Hohlraumresonators verlief das Keramikrohr durch den Hohlraum eines als Kopplungsantenne fungierenden Innenleiterrohres. Das von einem Magnetron erzeugte Mikrowellenfeld mit einer Frequenz von 2,45 GHz wurde mittels der Kopplungsantenne in den Hohlraumresonator eingekoppelt (E₀₁-Hohlraumapplikator; Monomode).

Die Mikrowellenleistung wurde über die Versuchsdauer jeweils in der Art eingestellt, dass die gewünschte Temperatur des Reaktionsguts am Ende der Bestrahlungszone konstant gehalten wurde. Die in den Versuchsbeschreibungen genannten Mikrowellenleistungen repräsentieren daher den zeitlichen Mittelwert der eingestrahlten Mikrowellenleistung. Die Temperaturmessung des Reaktionsgemischs wurde direkt nach Verlassen der Reaktionszone (etwa 15 cm Strecke in einer isolierten Edelstahikapillare, Ø 1 cm) mittels Pt100 Temperatursensor vorgenommen. Vom Reaktionsgemisch nicht direkt absorbierte Mikrowellenenergie wurde an der der Kopplungsantenne entgegen liegenden Stirnseite des Hohlraumresonators reflektiert; die vom Reaktionsgemisch auch beim Rücklauf nicht absorbierte und in Richtung des Magnetrons zurück gespiegelte Mikrowellenenergie wurde mit Hilfe eines Prismensystems (Zirkulator) in ein Wasser enthaltendes Gefäß geleitet. Aus der Differenz zwischen eingestrahlter Energie und Aufheizung dieser Wasserlast wurde die in das Reaktionsgut eingetragene Mikrowellenenergie berechnet

Mittels einer Hochdruckpumpe und eines geeigneten Druckentlastungsventils wurde die Reaktionsmischung im Reaktionsrohr unter einen solchen Arbeitsdruck gesetzt, der ausreichte, um alle Edukte und Produkte bzw. Kondensationsprodukte stets im flüssigen Zustand zu halten. Die aus Fettsäure und Amin hergestellten Ammoniumsalze wurden mit einer konstanten Flussrate durch das Reaktionsrohr gepumpt und die Verweilzeit in der Bestrahlungszone durch Modifizierung der Strömungsgeschwindigkeit eingestellt.

Die Analytik der Produkte erfolgte mittels ¹H-NMR-Spektroskopie bei 500 MHz in CDCl₃. Die Bestimmung von Eisengehalten erfolgte mittels Atomabsorptionsspektroskopie.

### Beispiel 1: Herstellung von Cocosfettsäuremonoethanolamid

In einem 10 Liter Rührautoklaven der Firma Büchi wurden 5,1 kg geschmolzene Cocosfettsäure (25 mol) vorgelegt und langsam unter leichter Kühlung mit 1,5 kg Ethanolamin (25 mol) versetzt. In einer exothermen Reaktion bildete sich das Cocosfettsäureethanolammonium Salz.

Das so erhaltene Ammoniumsalz wurde in geschmolzenem Zustand bei 120 °C und einem Arbeitsdruck von 25 bar kontinuierlich mit einem Fluss von 5 l/h durch das Reaktionsrohr gepumpt und einer Mikrowellenleistung von 2,2 kW ausgesetzt, von denen 90 % vom Reaktionsgut absorbiert wurden. Die Verweilzeit des Reaktionsgemischs in der Bestrahlungszone betrug ca. 34 Sekunden. Am Ende des Reaktionsrohres hatte das Reaktionsgemisch eine Temperatur von 265 °C.

Es wurde ein Umsatz von 94 % d. Th. erreicht. Das Reaktionsprodukt war praktisch farblos. Nach destillativer Abtrennung von Reaktionswasser und überschüssigem Ethanolamin wurden 6,0 kg Cocosfettsäuremonoethanolamid mit einer Reinheit von 93,5 % erhalten. Das so erhaltene Cocosfettsäuremonoethanolamid enthielt insgesamt weniger als 1 mol-% Aminoester und Esteramid.

### Beispiel 2: Herstellung von N-(2-Hydroxyethyl)laurinsäureamid

In einem 10 Liter Rührautoklaven der Firma Büchi wurden 4,00 kg geschmolzene Laurinsäure (20 mol) vorgelegt und langsam unter Kühlung mit 1,34 kg Ethanolamin (22 mol) versetzt. In einer exothermen Reaktion bildete sich das Laurinsäure-monoethanolammonium Salz.

Das so erhaltene Ammoniumsalz wurde in geschmolzenem Zustand bei 120 °C und einem Arbeitsdruck von 25 bar kontinuierlich mit einem Fluss von 5 l/h durch das Reaktionsrohr gepumpt und einer Mikrowellenleistung von 2,2 kW ausgesetzt, von denen 92 % vom Reaktionsgut absorbiert wurden. Die Verweilzeit des Reaktionsgemischs in der Bestrahlungszone betrug ca. 34 Sekunden. Am Ende des Reaktionsrohres hatte das Reaktionsgemisch eine Temperatur von 270 °C.

Es wurde ein Umsatz von 96 % d. Th. erreicht. Das Reaktionsprodukt war leicht gelblich gefärbt. Nach destillativer Abtrennung von Reaktionswasser und überschüssigem Ethanolamin wurden 4,7 kg N-(2-Hydroxyethyl)laurinsäureamid mit einer Reinheit von 95 % erhalten. Das so erhaltene Laurinsäure-N-monoethanolamid enthielt insgesamt 1,5 mol-% Aminoester und Esteramid.

### Beispiel 3: Umsetzung von Laurinsäure mit 2-(2-Aminoethoxy)ethanol

In einem 10 Liter Rührautoklaven der Firma Büchi wurden 4,00 kg geschmolzene Laurinsäure (20 mol) vorgelegt und langsam unter leichter Kühlung mit 2,1 kg 2-(2-Aminoethoxy)ethanol (20 mol) versetzt. In einer exothermen Reaktion bildete sich das Ammoniumsalz.

Das so erhaltene Ammoniumsalz wurde in geschmolzenem Zustand bei 90 °C und einem Arbeitsdruck von 20 bar kontinuierlich mit einem Fluss von 4 l/h durch das Reaktionsrohr gepumpt und einer Mikrowellenleistung von 2,9 kW ausgesetzt, von denen 95 % vom Reaktionsgut absorbiert wurden. Die Verweilzeit des Reaktionsgemischs in der Bestrahlungszone betrug ca. 42 Sekunden. Am Ende des Reaktionsrohres hatte das Reaktionsgemisch eine Temperatur von 265 °C.

Es wurde ein Umsatz von 95 % d. Th. erreicht. Das Reaktionsprodukt war gelblich gefärbt. Nach destillativer Abtrennung des Reaktionswassers wurden 5,6 kg N-Lauroyl-2-(2-aminoethoxy)ethanolamid mit einer Reinheit von 94 % erhalten. Das so erhaltene N-Lauroyl-2-(2-aminoethoxy)ethanolamid enthielt weniger als 1 mol-% Aminoester und Esteramid.

### Beipiel 4: Herstellung von Bis-(2-Hydroxyethyl)ölsäureamid

In einem 10 Liter Rührautoklaven der Firma Büchi wurden 5,65 kg technische Ölsäure (20 mol) vorgelegt und langsam unter leichter Kühlung mit 2,1 kg Diethanolamin (20 mol) versetzt. In einer exothermen Reaktion bildete sich das Ölsäurediethanolammonium Salz.

Das so erhaltene Ammoniumsalz wurde in geschmolzenem Zustand bei 100 °C und einem Arbeitsdruck von 25 bar kontinuierlich mit einem Fluss von 9,3 l/h durch das Reaktionsrohr gepumpt und einer Mikrowellenleistung von 3,5 kW ausgesetzt, von denen 93 % vom Reaktionsgut absorbiert wurden. Die Verweilzeit des Reaktionsgemischs in der Bestrahlungszone betrug ca. 18 Sekunden. Am Ende des Reaktionsrohres hatte das Reaktionsgemisch eine Temperatur von 275 °C.

Es wurde ein Umsatz von 96 % d. Th. erreicht. Das Reaktionsprodukt war gelblich gefärbt. Nach destillativer Abtrennung von Reaktionswasser wurden 7,1 kg Bis-(2-Hydroxyethyl)ölsäureamid mit einer Reinheit von 95 % erhalten. Das so erhaltene Bis-(2-Hydroxyethyl)ölsäureamid enthielt insgesamt weniger als 1 mol-% Aminoester und Esteramid. Die ¹H-NMR-Signale der olefinischen Protonen des Produkts waren hinsichtlich Aufspaltungsmuster und Integral gegenüber der eingesetzten Ölsäure unverändert.

### Beispiel 5: Herstellung von Cocosfettsäurediethanolamid

In einem 10 Liter Rührautoklaven der Firma Büchi wurden 5,1 kg geschmolzene Cocosfettsäure (25 mol) vorgelegt und langsam unter leichter Kühlung mit 2,6 kg Diethanolamin (25 mol) versetzt. In einer exothermen Reaktion bildete sich das Cocosfettsäurediethanolammonium Salz.

Das so erhaltene Ammoniumsalz wurde in geschmolzenem Zustand bei 110 °C und einem Arbeitsdruck von 25 bar kontinuierlich mit einem Fluss von 5 l/h durch das Reaktionsrohr gepumpt und einer Mikrowellenleistung von 2,0 kW ausgesetzt, von denen 92 % vom Reaktionsgut absorbiert wurden. Die Verweilzeit des Reaktionsgemischs in der Bestrahlungszone betrug ca. 34 Sekunden. Am Ende des Reaktionsrohres hatte das Reaktionsgemisch eine Temperatur von 270 °C.

Es wurde ein Umsatz von 92 % d. Th. erreicht. Das Reaktionsprodukt war praktisch farblos. Nach destillativer Abtrennung von Reaktionswasser und überschüssigem Diethanolamin wurden 7,1 kg Cocosfettsäuremonoethanolamid mit einer Reinheit von 91 % erhalten. Das so erhaltene Cocosfettsäuremonoethanolamid enthielt insgesamt weniger als 1 mol% Aminoester und Esteramid.

## Patentansprüche

1. Kontinuierliches Verfahren zur Herstellung von Fettsäurealkanolamiden, indem mindestens eine Fettsäure der Formel I
R³-COOH (I)
worin R³ für einen gegebenenfalls substituierten aliphatischen Kohlenwasserstoffrest mit 5 bis 50 C-Atomen steht,
mit mindestens einem Alkanolamin der Formel II
HNR¹R² (II)
worin
R¹ für einen mindestens eine Hydroxylgruppe tragenden Kohlenwasserstoffrest mit 1 bis 50 Kohlenstoffatomen steht, oder für einen linearen oder verzweigten Alkylrest, welcher durch Heteroatome unterbrochen sein kann und eine oder mehrere Hydroxylgruppen trägt, steht, oder wobei R¹ für eine Gruppe der Formel III
-(B-O)ₘ-H (III)
steht, worin
B für einen Alkylenrest mit 2 bis 10 C-Atomen und
m für eine Zahl von 1 bis 500 stehen, und
R² für Wasserstoff, R¹, einen Kohlenwasserstoffrest mit 1 bis 50 C-Atomen, eine heteroaromatische Gruppe mit 5 bis 12 Ringgliedern, wobei die Kohlenwasserstoffreste Heteroatome enthalten können sowie gegebenenfalls Substituenten tragen, oder für eine Gruppe der Formel IV
-(B-O)ₘ-R⁵ (IV)
steht, worin
B für einen Alkylenrest mit 2 bis 10 C-Atomen,
m für eine Zahl von 1 bis 500, und
R⁵ für einen Kohlenwasserstoffrest mit 1 bis 24 C-Atomen steht,
zu einem Ammoniumsalz umgesetzt wird und dieses Ammoniumsalz nachfolgend unter Mikrowellenbestrahlung in einem Reaktionsrohr, dessen Längsachse sich in der Ausbreitungsrichtung der Mikrowellen eines Monomode-Mikrowellenapplikators befindet, zum Fettsäurealkanolamid umgesetzt wird, wobei die Bestrahlung des Salzes mit Mikrowellen in einem weitgehend mikrowellentransparenten Reaktionsrohr innerhalb eines mit einem Mikrowellengenerator über Wellenleiter verbundenen Hohlleiters erfolgt.

2. Verfahren nach Anspruch 1, bei dem der Mikrowellenapplikator als Hohlraumresonator ausgestaltet ist.

3. Verfahren nach Anspruch 1 oder 2, bei dem der Mikrowellenapplikator als Hohlraumresonator vom Reflexionstyp ausgestaltet ist.

4. Verfahren nach einem oder mehreren der Ansprüche 1 bis 3, bei dem das Reaktionsrohr axial mit einer zentralen Symmetrieachse des Hohlleiters fluchtet.

5. Verfahren nach einem oder mehreren der Ansprüche 1 bis 4, bei dem die Bestrahlung des Salzes in einem Hohlraumresonator mit koaxialem Übergang der Mikrowellen erfolgt.

6. Verfahren nach einem oder mehreren der Ansprüche 1 bis 5, bei dem der Hohlraumresonator im E₀₁ₙ-Mode betrieben wird, wobei n eine ganze Zahl von 1 bis 200 ist.

7. Verfahren nach einem oder mehreren der Ansprüche 1 bis 6, bei dem R³ ein unsubstituierter Alkylrest mit 5 bis 50 C-Atomen ist.

8. Verfahren nach einem oder mehreren der Ansprüche 1 bis 6, bei dem R³ ein Kohlenwasserstoffrest mit 5 bis 50 C-Atomen ist, der einen oder mehrere Substituenten ausgewählt aus Halogenatomen, halogenierten Alkylresten, C₁-C₅-Alkoxy-, Poly(C₁-C₅-Alkoxy)-, Poly(C₁-C₅-Alkoxy)alkyl-, Carboxyl-, Ester-, Amid-, Cyano-, Nitril-, Nitro-, Sulfonsäure- und Arylgruppen mit 5 bis 20 Kohlenstoffatomen trägt, wobei die C₅-C₂₀-Arylgruppen Substituenten ausgewählt aus Halogenatomen, halogenierten Alkylresten, C₁-C₂₀-Alkyl-, C₂-C₂₀-Alkenyl-, C₁-C₅-Alkoxy -, Ester-, Amid-, Cyano-, Nitril-, und Nitrogruppen tragen können.

9. Verfahren nach einem oder mehreren der Ansprüche 1 bis 8, bei dem R³ 5 bis 30 Kohlenstoffatome umfasst.

10. Verfahren nach einem oder mehreren der Ansprüche 1 bis 9, bei dem R³ eine oder mehrere Doppelbindungen umfasst.

11. Verfahren nach einem oder mehreren der Ansprüche 1 bis 10, bei dem R¹ 2 bis 20 Kohlenstoffatome trägt.

12. Verfahren nach einem oder mehreren der Ansprüche 1 bis 11, bei dem R² für Wasserstoff steht.

13. Verfahren nach einem oder mehreren der Ansprüche 1 bis 11, bei dem R² für Alkylreste mit 1 bis 20 C-Atomen oder für Alkenylreste mit 2 bis 20 C-Atomen steht.

14. Verfahren nach einem oder mehreren der Ansprüche 1 bis 13, bei dem die Mikrowellenbestrahlung bei Temperaturen zwischen 150 und 500 °C durchgeführt wird.

15. Verfahren nach einem oder mehreren der Ansprüche 1 bis 14, bei dem die Mikrowellenbestrahlung bei Drücken oberhalb des Atmosphärendrucks erfolgt

## Claims

1. A continuous process for preparing fatty acid alkanolamides by reacting at least one fatty acid of the formula I
R³-COOH (I)
in which R³ is an optionally substituted aliphatic hydrocarbon radical having 5 to 50 carbon atoms
with at least one alkanolamine of the formula II
HNR¹R² (II)
in which
R¹ is a hydrocarbon radical bearing at least one hydroxyl group and having 1 to 50 carbon atoms, or a linear or branched alkyl radical which may be interrupted by heteroatoms and bears one or more hydroxyl groups, or in which R¹ is a group of the formula III
-(B-O)ₘ-H (III)
in which
B is an alkylene radical having 2 to 10 carbon atoms and
m is from 1 to 500, and
R² is hydrogen, R¹, a hydrocarbon radical having 1 to 50 carbon atoms, a heteroaromatic group having 5 to 12 ring members, wherein the hydrocarbon radicals may comprise heteroatoms and optionally bear substituents, or is a group of the formula IV
-(B-O)ₘ-R⁵ (IV)
in which
B is an alkylene radical having 2 to 10 carbon atoms,
m is from 1 to 500, and
R⁵ is a hydrocarbon radical having 1 to 24 carbon atoms,
to give an ammonium salt and then converting this ammonium salt to the fatty acid alkanolamide under microwave irradiation in a reaction tube whose longitudinal axis is in the direction of propagation of the microwaves from a monomode microwave applicator, wherein the salt is irradiated with microwaves in a substantially microwave-transparent reaction tube within a hollow conductor connected via waveguides to a microwave generator.

2. The process as claimed in claim 1, in which the microwave applicator is configured as a cavity resonator.

3. The process as claimed in claim 1 or 2, in which the microwave applicator is configured as a cavity resonator of the reflection type.

4. The process as claimed in one or more of claims 1 to 3, in which the reaction tube is aligned axially with a central axis of symmetry of the hollow conductor.

5. The process as claimed in one or more of claims 1 to 4, in which the salt is irradiated in a cavity resonator with a coaxial transition of the microwaves.

6. The process as claimed in one or more of claims 1 to 5, in which the cavity resonator is operated in E₀₁ₙ mode where n is an integer from 1 to 200.

7. The process as claimed in one or more of claims 1 to 6, in which R³ is an unsubstituted alkyl radical having 5 to 50 carbon atoms.

8. The process as claimed in one or more of claims 1 to 6, in which R³ is a hydrocarbon radical which has 5 to 50 carbon atoms and bears one or more substituents selected from halogen atoms, halogenated alkyl radicals, C₁-C₅-alkoxy, poly(C₁-C₅-alkoxy), poly(C₁-C₅-alkoxy)alkyl, carboxyl, ester, amide, cyano, nitrile, nitro, sulfo and aryl groups having 5 to 20 carbon atoms, where the C₅-C₂₀-aryl groups may bear substituents selected from halogen atoms, halogenated alkyl radicals, C₁-C₂₀-alkyl, C₂-C₂₀-alkenyl, C₁-C₅-alkoxy, ester, amide, cyano, nitrile and nitro groups.

9. The process as claimed in one or more of claims 1 to 8, in which R³ comprises 5 to 30 carbon atoms.

10. The process as claimed in one or more of claims 1 to 9, in which R³ comprises one or more double bonds.

11. The process as claimed in one or more of claims 1 to 10, in which R¹ bears 2 to 20 carbon atoms.

12. The process as claimed in one or more of claims 1 to 11, in which R² is hydrogen.

13. The process as claimed in one or more of claims 1 to 11, in which R² represents alkyl radicals having 1 to 20 carbon atoms or alkenyl radicals having 2 to 20 carbon atoms.

14. The process as claimed in one or more of claims 1 to 13, in which the microwave irradiation is performed at temperatures between 150 and 500°C.

15. The process as claimed in one or more of claims 1 to 14, in which the microwave irradiation is performed at pressures above atmospheric pressure.

## Revendications

1. Procédé continu pour la fabrication d'alcanolamides d'acides gras, selon lequel au moins un acide gras de formule I
R³-COOH (I)
dans laquelle R³ représente un radical hydrocarboné aliphatique éventuellement substitué de 5 à 50 atomes C,
est mis en réaction avec au moins une alcanolamine de formule II
HNR¹R² (II)
dans laquelle
R¹ représente un radical hydrocarboné portant au moins un groupe hydroxyle de 1 à 50 atomes de carbone, ou un radical alkyle linéaire ou ramifié, qui peut être interrompu par des hétéroatomes et qui porte un ou plusieurs groupes hydroxyle, ou R¹ représente un groupe de formule III
-(B-O)ₘ-H (III)
dans laquelle
B représente un radical alkylène de 2 à 10 atomes C et
m représente un nombre de 1 à 500, et
R² représente l'hydrogène, R¹, un radical hydrocarboné de 1 à 50 atomes C, un groupe hétéroaromatique de 5 à 12 éléments de cycle, les radicaux hydrocarbonés pouvant contenir des hétéroatomes et portant éventuellement des substituants, ou un groupe de formule IV
-(B-O)ₘ-R⁵ (IV)
dans laquelle
B représente un radical alkylène de 2 à 10 atomes C, m représente un nombre de 1 à 500 et
R⁵ représente un radical hydrocarboné de 1 à 24 atomes C,
pour former un sel d'ammonium, et ce sel d'ammonium est ensuite transformé en l'alcanolamide d'acide gras par exposition à des micro-ondes dans un tube de réaction, dont l'axe longitudinal se trouve dans la direction de propagation des micro-ondes d'un applicateur monomodal de micro-ondes, l'exposition du sel à des micro-ondes ayant lieu dans un tube de réaction essentiellement transparent aux micro-ondes dans un conducteur creux connecté avec un générateur de micro-ondes par un guide d'ondes.

2. Procédé selon la revendication 1, dans lequel l'applicateur de micro-ondes est configuré sous la forme d'une cavité résonnante.

3. Procédé selon la revendication 1 ou 2, dans lequel l'applicateur de micro-ondes est configuré sous la forme d'une cavité résonnante de type à réflexion.

4. Procédé selon une ou plusieurs des revendications 1 à 3, dans lequel le tube de réaction est aligné axialement avec un axe de symétrie central du conducteur creux.

5. Procédé selon une ou plusieurs des revendications 1 à 4, dans lequel l'exposition du sel a lieu dans une cavité résonnante avec transition coaxiale des micro-ondes.

6. Procédé selon une ou plusieurs des revendications 1 à 5, dans lequel la cavité résonnante est exploitée en mode E₀₁ₙ, n étant un nombre entier de 1 à 200.

7. Procédé selon une ou plusieurs des revendications 1 à 6, dans lequel R³ est un radical alkyle non substitué de 5 à 50 atomes C.

8. Procédé selon une ou plusieurs des revendications 1 à 6, dans lequel R³ est un radical hydrocarboné de 5 à 50 atomes C, qui porte un ou plusieurs substituants choisis parmi les atomes d'halogène, les radicaux alkyle halogénés, les groupes alcoxy en C₁-C₅, poly (alcoxy en C₁-C₅), poly (alcoxy en C₁-C₅)alkyle, carboxyle, ester, amide, cyano, nitrile, nitro, acide sulfonique et aryle de 5 à 20 atomes de carbone, les groupes aryle en C₅-C₂₀ pouvant porter des substituants choisis parmi les atomes d'halogène, les radicaux alkyle halogénés, les groupes alkyle en C₁-C₂₀, alcényle en C₂-C₂₀, alcoxy en C₁-C₅, ester, amide, cyano, nitrile et nitro.

9. Procédé selon une ou plusieurs des revendications 1 à 8, dans lequel R³ comprend 5 à 30 atomes de carbone.

10. Procédé selon une ou plusieurs des revendications 1 à 9, dans lequel R³ comprend une ou plusieurs doubles liaisons.

11. Procédé selon une ou plusieurs des revendications 1 à 10, dans lequel R¹ porte 2 à 20 atomes de carbone.

12. Procédé selon une ou plusieurs des revendications 1 à 11, dans lequel R² représente l'hydrogène.

13. Procédé selon une ou plusieurs des revendications 1 à 11, selon lequel R² représente des radicaux alkyle de 1 à 20 atomes C ou des radicaux alcényle de 2 à 20 atomes C.

14. Procédé selon une ou plusieurs des revendications 1 à 13, dans lequel l'exposition à des micro-ondes est réalisée à des températures comprises entre 150 et 500 °C.

15. Procédé selon une ou plusieurs des revendications 1 à 14, dans lequel l'exposition à des micro-ondes est réalisée à des pressions supérieures à la pression atmosphérique.
